Europäisches Patentamt

European Patent Office

Office européen des brevets

(19)

(11) Numéro de publication: **0 204 640**
**A1**

(12) **DEMANDE DE BREVET EUROPEEN**

(21) Numéro de dépôt: **86401232.3**

(22) Date de dépôt: **06.06.86**

(51) Int. Cl.⁴: **A61N 1/40**

(30) Priorité: **07.06.85 FR 8508641**

(43) Date de publication de la demande:
**10.12.86 Bulletin 86/50**

(84) Etats contractants désignés:
**DE IT SE**

(71) Demandeur: **C.G.R. MeV**
**551, route de la Minière**
**F-78530 Buc(FR)**

(72) Inventeur: **Azam, Guy**
**THOMSON-CSF SCPI 19, avenue de Messine**
**F-75008 Paris(FR)**
Inventeur: **Convert, Guy**
**THOMSON-CSF SCPI 19, avenue de Messine**
**F-75008 Paris(FR)**
Inventeur: **Cosset, Jean-Marc**
**THOMSON-CSF SCPI 19, avenue de Messine**
**F-75008 Paris(FR)**
Inventeur: **Dufour, Jacques**
**THOMSON-CSF SCPI 19, avenue de Messine**
**F-75008 Paris(FR)**
Inventeur: **Mabire, Jean-Pierre**
**THOMSON-CSF SCPI 19, avenue de Messine**
**F-75008 Paris(FR)**

(74) Mandataire: **Grynwald, Albert et al**
**THOMSON-CSF SCPI 19, avenue de Messine**
**F-75008 Paris(FR)**

(54) **Dispositif de traitement par hyperthermie.**

(57) La présente invention concerne un dispositif de traitement par hyperthermie, comportant des sondes unipolaires (S1, S2) destinées à être disposées dans une zone (8) à traiter d'un patient, et comportant des tubes vecteurs (V1, V2), destinés à être implantés à demeure dans ladite zone (8) à traiter et à contenir, à chaque séance d'hyperthermie, lesdites sondes unipolaires (S1, S2).

FIG_1

GENERATEUR

$G_1$

## DISPOSITIF DE TRAITEMENT PAR HYPERTHERMIE

L'invention concerne un dispositif de traitement par hyperthermie, permettant de chauffer une région malade d'un patient par la dissipation d'une énergie électrique appliquée selon un champ électrique grâce à des électrodes unipolaires disposées au voisinage de la région malade ou dans cette dernière. L'invention est remarquable en ce qu'elle permet de réduire considérablement les effets, traumatisants pour le patient, procurés par de tels traitements.

L'hyperthermie est un procédé connu, qui consiste à chauffer des tissus biologiques vivants à des températures sensiblement supérieures à leur température normale, et qui est utilisé dans le traitement de diverses maladies et notamment en cancérothérapie. Dans l'exemple de cette dernière application, il est souhaitable de chauffer les tissus à traiter à des températures de l'ordre de 44°C à 45°C, tout en évitant autant que possible, une élévation notable de la température des tissus sains environnants.

Cette condition soulève un problème qui réside dans la localisation correcte de la zone chauffée. Dans certaines configurations que peuvent présenter les tissus malades, cette localisation correcte du chauffage est impossible à obtenir avec des électrodes situées à l'extérieur du corps du patient. Aussi dans de nombreux cas, le traitement par hyperthermie est réalisé grâce à des électrodes implantées directement dans ou autour de la zone à traiter, de manière à mieux circonscrire la zone chauffée.

De telles sondes peuvent être soit du type bipolaire ou du type unipolaire. Dans le cas des sondes bipolaires, l'énergie électrique à haute fréquence délivrée par un générateur, sous la forme d'une tension, peut être appliquée à une unique sonde de ce type, pour chauffer la région dans laquelle elle est implantée. Dans le cas des sondes unipolaires, la tension à haute ou moyenne fréquence délivrée par le générateur est appliquée à deux sondes unipolaires distinctes, chacune des deux sondes étant reliée à l'un des pôles de sortie du générateur, la zone chauffée étant alors principalement établie entre les deux sondes unipolaires de type implantable.

L'implantation d'une sonde bipolaire ou unipolaire dans une région malade, représente pour un patient une opération qui peut s'accompagner d'une douleur non négligeable, et qui est particulièrement traumatisante en ce que les traitements par hyperthermie pour une zone à traiter donnée, doivent généralement être répétées à des fréquences variables et sur des périodes de temps comprises entre quelques jours et quelques dizaines de jours.

Un but général de la présente invention est de réaliser une structure d'implantation des sondes, unipolaires ou bipolaires, permettant de diminuer autant que possible, les désagréments subis par le patient.

Plus précisément, un des buts de l'invention est de supprimer les désagréments dus à la répétition des implantations des sondes.

Il est à remarquer en outre que durant le traitement, certains tissus de la zone à traiter peuvent accuser des élévations de température différentes, ceci pouvant conduire au cours du traitement à modifier l'implantation des sondes. Aussi, un des buts de l'invention est également de permettre de modifier la configuration de la zone chauffée, sans exiger une modification de l'implantation des sondes.

Selon l'invention, un dispositif de traitement par hyperthermie comportant, au moins un générateur délivrant une énergie électrique alternative, ladite énergie électrique étant appliquée, selon un champ électrique, à une zone à traiter d'un patient grâce à deux électrodes unipolaires reliées audit générateur, est caractérisé en ce qu'il comporte en outre au moins un second générateur synchronisé en fréquence avec le premier générateur, et relié à une troisième et quatrième électrodes unipolaires coopérant avec les deux premières électrodes unipolaires pour chauffer la zone à traiter.

L'invention sera mieux comprise grâce à la description qui suit faite à titre d'exemple non limitatif, et à l'aide des quatres figures annexées, parmi lesquelles :

-la figure 1 illustre l'implantation de sondes unipolaires dans une zone à traiter,

-la figure 2 montre des détails d'un tube vecteur montré dans la figure 1 et destiné, à contenir une sonde unipolaire ;

-la figure 3 montre schématiquement une version préférée, à au moins deux générateurs, d'un dispositif de traitement selon l'invention permettant de modifier la forme de distribution des courants dans une zone à traiter ;

-la figure 4 illustre schématiquement une modification de la forme de distribution des courants par rapport à la figure 3.

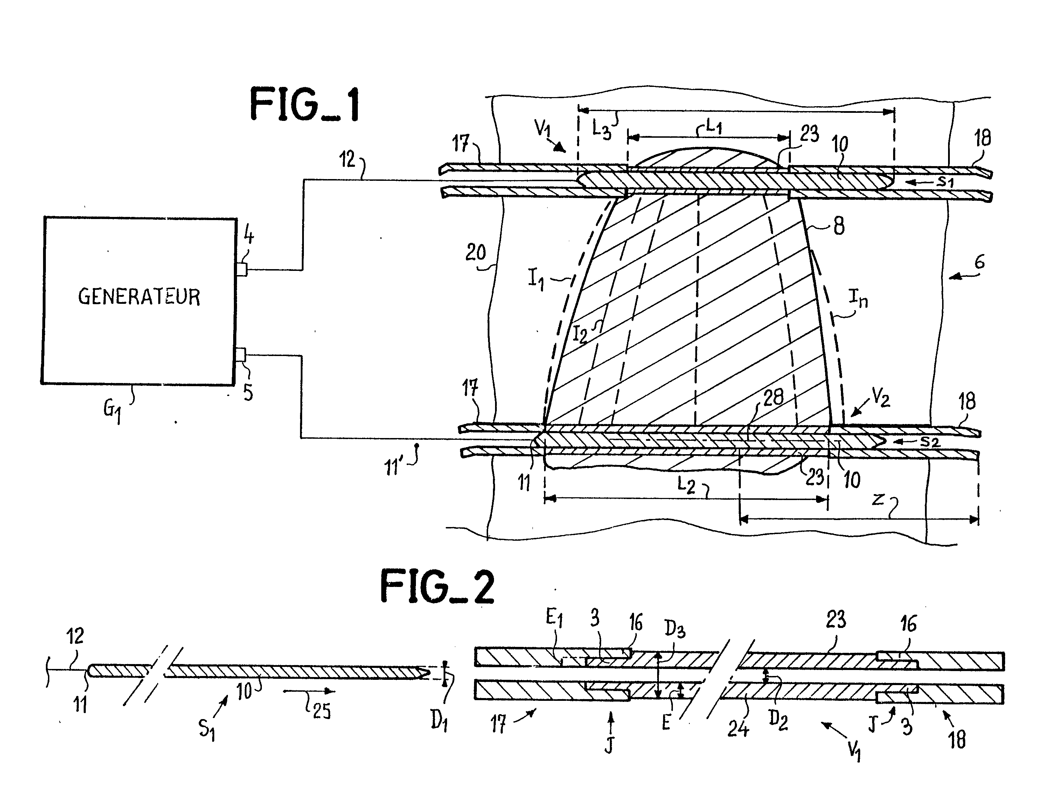

La figure 1 illustre un dispositif de traitement 1 par l'hyperthermie, et un exemple d'implantation de sondes unipolaires S1, S2.

Le dispositif de traitement comporte un générateur G1 du type fonctionnant à moyenne ou haute fréquence (de l'ordre de 100 KHZ à plusieurs MHZ), et de puissance moyenne (de l'ordre de quelques dizaines de Watt à quelques centaines de Watt). Le générateur G1 délivre, entre une première et une seconde bornes de sortie 4, 5, une énergie électrique alternative destinée à être dissipée dans le corps 6 (partiellement représenté) d'un patient, afin d'élever la température d'une zone 8 à traiter par hyperthermie. L'énergie électrique délivrée par le générateur G1, sous la forme d'une tension (non représentée) par exemple, est appliquée aux sondes unipolaires S1, S2, la première sonde unipolaire S1 étant à cet effet reliée à la première borne de sortie 4 et la seconde sonde unipolaire S2 étant reliée à la seconde borne de sortie 5. Dans l'exemple non limitatif décrit, les sondes unipolaires S1, S2 sont toutes deux du type implantables, c'est-à-dire constituées par une aiguille métallique 10 dont une extrémité 11 est connectée à un fil conducteur électrique 12 classique, par lequel elles sont reliées au générateur G1 ; mais il est également connu dans l'art antérieur, et ceci peut également être appliqué dans le cadre de l'invention, d'utiliser une unique sonde implantable S1, S2 du type unipolaire coopérant avec une électrode unipolaire (non représentée) destinée à être placée à l'extérieur du corps 6 du patient.

Dans l'exemple non limitatif décrit, le dispositif de traitement 1 comporte en outre des tubes vecteurs V1, V2 prévus pour être implantés dans la zone 8 à traiter ou à son voisinage, ainsi qu'il est représenté dans l'exemple non limitatif de la figure 1, et à y être laissés à demeure, c'est-à-dire sur une période de temps contenant plusieurs séances de traitement par l'hyperthermie. Les tubes vecteurs V1, V2 sont destinés à contenir, durant chaque séance de traitement, une sonde unipolaire S1, S2 introduite dans un tube vecteur V1, V2 par une première ou une seconde extrémité 17, 18 de ce dernier, au moins une de ces extrémités 17, 18 devant à cet effet être à l'extérieur du corps 6 du patient c'est-à-dire en dépassement par rapport à la peau 20 du patient.

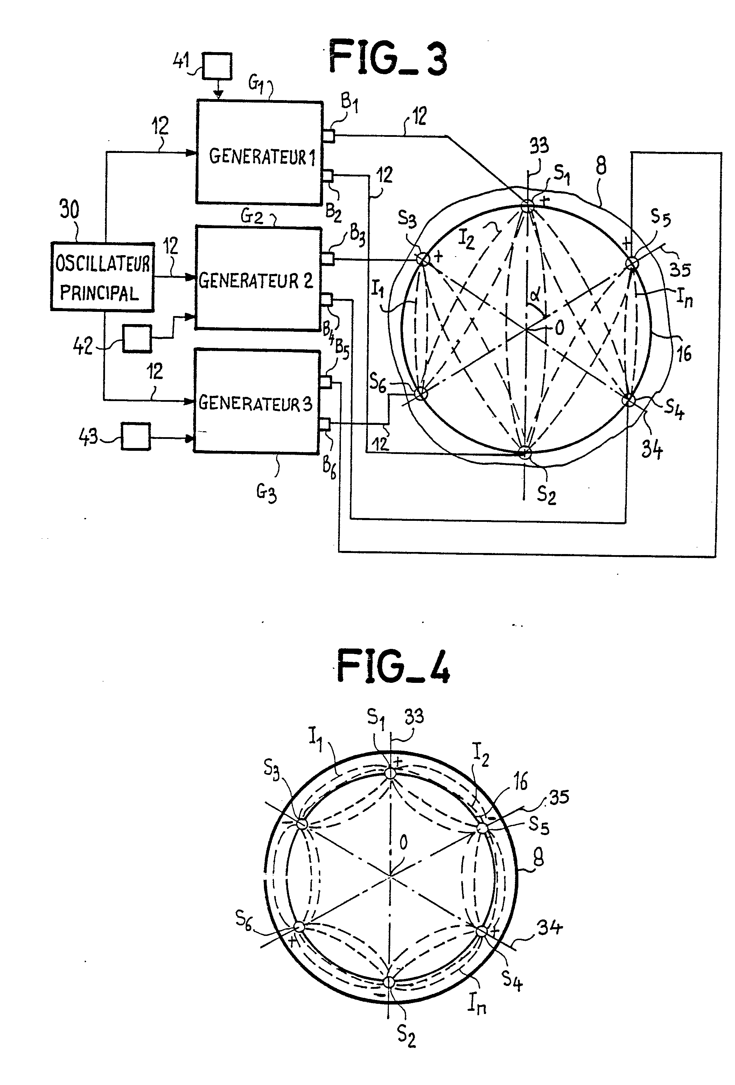

La figure 2 montre de manière plus claire, à titre d'exemple non limitatif, un tube vecteur V1 et une sonde unipolaire S1. Les première et seconde extrémités 17, 18 du tube vecteur V1, dont au moins une est nécessaire, sont réalisées, en un matériau électriquement isolant et souple.

La première extrémité tubulaire 17 est prolongée par un tube métallique 23, communiquant avec l'extrémité tubulaire 17, de manière qu'une sonde unipolaire S1 puisse être introduite dans le sens montré par la première flèche 25 par exemple, jusque dans le tube métallique 23. Dans l'exemple non limitatif décrit, l'aiguille métallique 10 de la sonde unipolaire S1 a un diamètre D1 sensiblement inférieur à un diamètre intérieur D2 du tube métallique 23 ; ceci permettant d'assurer de manière simple, un contact électrique entre l'aiguille métallique 10 et le tube métallique 23 quand l'aiguille est introduite au moins partiellement dans ce dernier. En supposant que l'aiguille 10 ait de manière classique un diamètre D1 de l'ordre de 0,8 mm, et que le diamètre intérieur D2 du tube métallique 23 soit de l'ordre de 0,9 à 1 mm par exemple, afin de permettre le passage de l'aiguille 10 et assurer le contact électrique, et que d'autre part la paroi 24 du tube métallique 23 comporte une épaisseur E de l'ordre de 0,1 à 0,15 mm, un diamètre extérieur D3 du tube métallique 23 est de l'ordre de 1,1 à 1,2 mm. L'assemblage entre le tube métallique 23 et les extrémités tubulaires 17, 18 peut être réalisé par l'homme du métier de différentes manières, comme par exemple représenté à la figure 2, où le tube métallique 23 comporte du côté de sa jonction J avec les extrémités tubulaires 17, 18, une partie d'extrémité 3 où la paroi 24 comporte une épaisseur E1 plus faible, de manière que l'extrémité tubulaire 17 puisse être enfoncée sur cette partie d'extrémité 3 sans apporter de surépaisseur notable par rapport au diamètre extérieur D3 ; la partie d'extrémité 3 comportant ainsi un surgainage 16 isolant électriquement.

L'implantation du tube vecteur V1 dans la zone 8 à traiter, peut s'effectuer en utilisant une aiguille creuse (non représentée) du type appelé gouttière vectrice par exemple dont l'emploi dans le domaine médical est courant, notamment pour l'implantation de diverses sondes dans des tissus vivants ; une telle gouttière vectrice étant également décrite dans un brevet français publié sous le n° 2 421 628. Le tube vecteur V1 peut être placé dans la gouttière vectrice, laquelle est ensuite introduite dans la zone 8 à traiter à l'emplacement voulu ; la gouttière vectrice est ensuite retirée avec précaution en faisant coulisser celle-ci sur le tube vecteur V1 qui reste en place.

En référence à nouveau à la figure 1, les tubes vecteurs V1, V2 étant en place, chaque sonde unipolaire S1, S2 est introduite dans un tube vecteur V1, V2. Les sondes unipolaires S1, S2 étant reliées au générateur 7, et celui-ci étant en fonctionnement, un champ électrique représenté sur la figure 1 par des lignes de champ $I_1$, $I_2$, ..., $I_n$ est établi entre les sondes unipolaires S1, S2 par l'intermédiaire du tube vecteur V1, V2, afin d'amener une élévation de la température de la zone à traiter 8 soumise au champ électrique. Un avantage de cette disposition réside en ce qu'elle permet de conférer aux tubes vecteurs V1, V2 des longueurs actives respectivement L1, L2 entre lesquelles est établi un champ électrique, indépendamment des longueurs L3 des sondes unipolaires S1, S2 ; ceci permettant de mieux circonscrire la zone 8 soumise au champ électrique. En effet, pour des fréquences inférieures à quelques MHZ, des épaisseurs de matériaux isolants de l'ordre du dixième de mm suffisent à empêcher l'établissement du courant électrique. Dans l'exemple non limitatif décrit, les longueurs actives L1, L2 correspondent à la longueur des tubes métalliques 23 mis à nu, c'est-à-dire non surgainés par les extrémités tubulaires 17, 18 en matériau isolant. Ceci permet notamment de conférer aux longueurs actives L1, L2 des dimensions éventuellement différentes, compatibles avec la géométrie de la zone 8 à traiter, tout en utilisant des sondes unipolaires S1, S2 ayant des aiguilles métalliques 10 d'une même longueur L3.

Les extrémités tubulaires 17, 18 assurent en outre un rôle de protection vis à vis de la peau 20, laquelle est particulièrement sensible aux élévations de température.

Un autre avantage très important pour le confort du patient, est apporté par la souplesse des extrémités tubulaires 17, 18, qui leur permet des mouvements relatifs par rapport à un axe longitudinal 28 des sondes unipolaires S1, S2, cette souplesse leur permettant de suivre des mouvements de la peau dus par exemple à de légers mouvements du patient, permettant ainsi d'éviter à ce dernier de ressentir des tiraillements douloureux.

Dans l'exemple non limitatif de la figure 1, deux tubes vecteurs V1, V2 ont été représentés, mais un plus grand nombre n de tels tubes vecteurs V1, V2, ..., Vn peut être implanté à demeure, notamment quand un plus grand nombre de sondes unipolaires S1, S2 sont utilisées avec plusieurs générateurs (non représentés sur la figure 1) dans le cas où le praticien désire modifier la configuration du chauffage d'une zone 8 traitée. Une telle modification peut être souhaitable d'une séance de traitement par hyperthermie à une séance suivante, ou même au cours d'une même séance, dans des cas par exemple où des tissus - (non représentés) d'une zone à traiter 8 ont des sensibilités différentes au champ électrique. La modification de la zone chauffée peut alors être réalisée en inversant les deux sondes unipolaires d'un même générateur par rapport aux tubes vecteurs dans lesquels ces sondes étaient placées, dans le cas où au moins deux générateurs alimentent chacun deux sondes unipolaires.

La figure 3 illustre schématiquement une version préférée du dispositif du traitement 1 selon l'invention, qui permet de réaliser des modifications de la zone chauffée ainsi que ci-dessus mentionnées, sans exiger ni la modification de position d'implantation de tubes vecteurs V1, V2, ... déjà implantés (non représentés sur la figure 3 pour plus de clarté), ni la modification de position des sondes unipolaires. Il est à remarquer que cette version de l'invention est également applicable dans le cas d'une utilistion classique de sondes unipolaires implantables, c'est-à-dire sans utiliser les tubes vecteurs V1, V2, ..., V3.

Dans cette version préférée, le dispositif de traitement 1 doit comporter, en plus du premier générateur G1, au moins un générateur supplémentaire G2, c'est-à-dire au moins deux générateurs G1, G2 synchronisés en fréquence.

Aussi, dans l'exemple non limitatif décrit, le dispositif de traitement 1 comporte, outre le premier générateur G1, un second et un troisième générateur G2, G3, sensiblement d'un même type que le premier générateur G1 ; un nombre N de générateur, supérieur à 3 pouvant également être utilisés. Dans l'exemple non limitatif décrit, le dispositif de traitement 1 comporte en outre un oscillateur principal 30 relié par des liaisons 31 à chacun des générateurs G1, G2, G3 afin que ces derniers fonctionnent à une même fréquence F1 et selon une même phase.

La zone à traiter 8 est représentée sur la figure 3 par une vue en coupe selon un plan perpendiculaire à l'axe longitudinal 28 montré sur la figure 1. Dans l'exemple décrit, six sondes unipolaires S1, S2, ..., S6 sont, soit implantées directement dans la zone à traiter 8, soit placées chacune, ainsi que dans l'exemple précédent, dans un tube vecteur préalablement implanté ; les sondes unipolaires S1, ..., S6 étant montrées selon leur section, elles sont représentées sur la figure 3 selon des cercles. Dans l'exemple non limitatif décrit, les sondes unipolaires S1, ..., S6 sont disposées dans la zone 8 à traiter selon sensiblement un cercle 16 ayant un centre O, par lequel passe trois axes 33, 34, 35 formant entre eux un angle $\alpha$ d'environ 30° ; deux sondes unipolaires S1, ..., S6 reliées à un même générateur G1, G2, G3 étant, dans l'exemple non limitatif décrit, disposées sur un même axe 33, 34, 35, mais de manière opposée par rapport au centre O. Chacune des sondes S1, ..., S6 est reliée par un conducteur électrique 12 à une des bornes de sortie B1, B2, ..., B6 d'un générateur G1, G2, G3 :

-les première et seconde sondes S1, S2, disposées sur le premier axe 33 sont reliées respectivement à la première et à la seconde borne B1, B2 du premier générateur G1 ;

-les troisième et quatrième sondes S3, S4 disposées selon le second axe 34, sont reliées respectivement à une troisième et quatrième bornes de sorties B3, B4 du second générateur G2 ;

-les cinquième et sixième sondes S5, S6 disposées sur le troisième axe 35, sont reliées respectivement à une cinquième et une sixième borne de sortie B5, B6 du troisième générateur G3.

Les trois générateurs G1, G2, G3 fonctionnant à une même fréquence F1, si on repère les polarités +, -(pour un instant donné) des bornes de sorties B1, B2, et B3, B4 et B5, B6 correspondant à chacun des générateurs G1, G2, G3, on peut placer les sondes unipolaires S1 à S6 correspondantes à une position voulue dans la zone 8 à traiter, telle que par exemple montrée à la figure 3, de manière à établir le champ électrique entre ces sondes unipolaires S1 à S6 selon une configuration souhaitée ; ce positionnement des sondes pouvant s'effectuer par implantation directe ou en plaçant chaque sonde dans un tube vecteur comme il a été précédemment expliqué.

Ainsi à supposer que les première, troisième et cinquième bornes de sortie B1, B3, B5 aient une polarité positive +, les seconde, quatrième et sixième bornes de sortie B2, B4, B6 auront une polarité négative -, ces polarités +, -se retrouvant respectivement au niveau des sondes unipolaires S1, S3, S5 et S2, S4, S6.

Le champ électrique représenté par les lignes de champ $I_1$ à $I_n$, sera dans ces conditions établi dans l'ensemble de la zone 8 à traiter.

Ainsi qu'il a déjà été mentionné, il peut être souhaitable de modifier la distribution du champ électrique c'est-à-dire la configuration de la zone chauffée. Ceci est possible dans l'invention, sans causer de désagréments supplémentaires au patient, soit en substituant les sondes unipolaires S1, ..., S6 entre elles, par rapport aux tubes vecteurs, dans le cas où ceux-ci sont utilisés et comme il a été précédemment mentionné, soit en modifiant la polarité +, -respective des sondes S1, à S6, en agissant au niveau d'au moins un des générateurs G1, G2, G3, aussi bien dans le cas où les sondes unipolaires S1 à S6 sont implantées directement dans la zone 8 à traiter, que dans le cas où elles sont disposées dans des tubes vecteurs. A cet effet, dans l'exemple non limitatif décrit, chacun des générateurs G1, G2, G3 comporte un dispositif inverseur respectivement 41, 42, 43, permettant d'inverser la polarité entre deux bornes de sortie B1, B2 et B3, B4 et B5, B6 d'un même générateur G1, G2, G3. Les dispositifs de commutation 41, 42, 43 peuvent consister en des moyens de commutation classiques permettant par exemple pour le premier générateur G1, d'inverser des connexions - (non représentées) établies entre les bornes de

sortie B1, B2 et des amplificateurs (non représentés) que comporte de manière classique chaque générateur G1, G2, G3 ; une telle inversion des polarités étant réalisable de différentes manières, toutes à la portée de l'homme du métier, et peut consister également dans une inversion des fils conducteurs 12 connectés aux bornes de sortie B1, B2.

On peut ainsi, par la modification de la polarité des sondes S1 à S6, obtenir une modification de la forme de la distribution du champ électrique, telle que par exemple représentée à la figure 4.

La figure 4 représente la zone 8 à traiter selon une vue semblable à celle de la figure 3, c'est-à-dire selon un plan perpendiculaire l'axe longitudinal 28 montré sur la figure 1 ; les sondes unipolaires S1 à S6 occupent une même position que sur la figure 3, mais comportent des polarités +, - différentes, de manière que ces polarités soient successivement positives + et négatives -. Le champ électrique, représenté par les lignes de champ $I_1$, ..., $I_n$ établies entre des sondes adjacentes de polarité +, -opposées, correspond alors sensiblement à une distribution circulaire le long du cercle 16, c'est-à-dire autour du centre O qui dans cette configuration n'est pas soumis au champ électrique.

D'autres configurations du chauffage de la zone 8 à traiter peuvent être obtenues, en modifiant soit la distribution des sondes S1, ..., S6 dans les tubes vecteurs V1, V2, ..., soit en modifiant la polarité positive + ou négative -conférées à chacune des sondes d'au moins un générateur.

Cette description constitue un exemple non limitatif d'un dispositif de traitement 1 par hyperthermie selon l'invention, qui permet de distribuer l'énergie servant à chauffer une zone à traiter selon une forme appropriée à cette zone, et éventuellement de modifier cette forme avec un minimum d'inconfort pour le patient, grâce à des moyens tels que, d'une part des tubes vecteurs $V_1$, $V_2$,...Vn dans lesquels les sondes unipolaires peuvent facilement être introduites ou retirées et d'autre part les moyens de commutation 41, 42, 43 qui peuvent être actionnés au niveau d'au moins un générateur G1, G2, G3.

**Revendications**

1. Dispositif de traitement par hyperthermie, comportant un générateur (G1) délivrant une énergie électrique alternative, ladite énergie électrique étant appliquée, selon un champ électrique (I1, ..., In), à une zone (8) à traiter d'un patient grâce à deux sondes unipolaires (S1, S2) du type sondes implantables, reliées audit générateur (G1), caractérisé en ce qu'il comporte en outre au moins un second générateur (G2), synchronisé en fréquence avec le premier générateur (G1), et relié à une troisième et quatrième sonde unipolaire (S3, S4) coopérant avec les deux premières sondes (S1, S2) pour chauffer ladite zone (8) à traiter et en ce qu'il comporte des moyens (41, 42, $V_1$, $V_2$) pour modifier la forme de la distribution dudit champ électrique ($I1, ..., In$).

2. Dispositif de traitement selon la revendication 1, caractérisé en ce que au moins un desdits générateurs (G1, G2) comporte des moyens de commutation (41, 42) pour inverser la polarité ( + , - ) appliquée aux deux sondes unipolaires (S1-S2, S3-S4) auxquelles il est relié, afin de modifier la distribution du champ électrique auquel est soumise ladite zone (8) à traiter.

3. Dispositif de traitement selon la revendication 1, caractérisé en ce que chacun desdits générateurs (G1, G2) comporte des moyens de commutation (41, 42) pour inverser la polarité ( + , - ) appliquée aux deux sondes unipolaires (S1-S2, S3-S4) auxquelles il est relié, afin de modifier la distribution du champ électrique auquel est soumise ladite zone (8) à traiter.

4. Dispositif de traitement selon l'une des revendications 1 à 3, caractérisé en ce qu'il comporte en outre un oscillateur principal (30) relié auxdits premier et second générateurs (G1, G2) pour les faire fonctionner à une même fréquence.

5. Dispositif de traitement selon la revendication 1, caractérisé en ce que les moyens pour modifier la forme de la distribution du champ électrique comportent au moins un tube vecteur - ($V_1$, $V_2$) destiné a être implanté dans la zone (8) à traiter et à contenir une sonde unipolaire ($S_1$, $S_2,...S_6$) durant le traitement, ledit tube vecteur ($V_1$, $V_2,...V_n$) comportant un tube métallique (23) dans lequel la sonde unipolaire ($S_1,...S_6$) est engagée, ledit tube métallique (23) étant en contact avec la zone à traiter et en contact électrique avec la sonde uniplaire ($S_1,...S_6$).

FIG_1

FIG_2

86401252.5

# FIG_3

41

$G_1$

12

GENERATEUR 1

$B_1$

12

$B_2$

12

$G_2$

$B_3$

30

OSCILLATEUR PRINCIPAL

12

GENERATEUR 2

42

$B_4$ $B_5$

12

GENERATEUR 3

43

$B_6$

$G_3$

33

$S_1$

8

$S_3$

$I_2$

$S_5$

35

$I_1$

$I_n$

$\alpha$

0

$S_6$

16

$S_2$

$S_4$

34

12

# FIG_4

$S_1$ 33

$I_1$

$I_2$

$S_3$

16

35

$S_5$

0

8

$S_6$

34

$S_4$

$I_n$

$S_2$

## RAPPORT DE RECHERCHE EUROPEENNE

Office européen
des brevets

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-4 448 198 (TURNER) <br> * Colonne 4, lignes 11-32; colonne 7, lignes 33-57 * | 1 | A 61 N 1/40 |
| A | | 2,4 | |
| | --- | | |
| A | US-A-4 346 715 (GAMMELL) <br> * Colonne 5, lignes 22-28; colonne 6, ligne 49 - colonne 7, ligne 38 * | 1-4 | |
| | --- | | |
| A | EP-A-0 128 076 (G.G.R. MEV) <br> * Page 4, lignes 18-33 * | 1-4 | |
| | ----- | | |

DOMAINES TECHNIQUES RECHERCHES (Int. Cl.4)

A 61 N

Le présent rapport de recherche a été établi pour toutes les revendications

| Lieu de la recherche <br> LA HAYE | Date d'achèvement de la recherche <br> 03-09-1986 | Examinateur <br> SIMON J.J.E. |
|---|---|---|